(19) Europäisches Patentamt
European Patent Office
Office européen des brevets

(11) EP 1 777 267 A2

(12) EUROPÄISCHE PATENTANMELDUNG

(43) Veröffentlichungstag:
**25.04.2007 Patentblatt 2007/17**

(51) Int Cl.:
***C09B 41/00*** *(2006.01)* ***C09D 11/00*** *(2006.01)*
***G03F 7/00*** *(2006.01)* ***G02B 5/22*** *(2006.01)*

(21) Anmeldenummer: **06014213.0**

(22) Anmeldetag: **08.07.2006**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **19.07.2005 DE 102005033582**

(71) Anmelder: **LANXESS Deutschland GmbH**
**51369 Leverkusen (DE)**

(72) Erfinder:
- **Feldhues, Ulrich, Dr.**
  **51465 Bergisch Gladbach (DE)**
- **Linke, Frank**
  **51069 Köln (DE)**
- **Pfützenreuter, Dirk**
  **51399 Burscheid (DE)**

(54) **Verfahren zur Herstellung von Metall-Verbindungen einer Azo-Verbindung unter Einsatz einer Umpumpung**

(57) Verfahren zur Herstellung von Metallverbindungen einer Azo-Verbindung, die in Form ihrer tautomeren Strukturen der Formel (I) entspricht

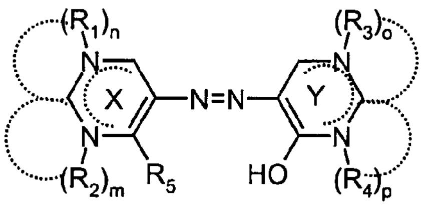

(I)

dadurch gekennzeichnet, dass die Herstellung unter Einsatz einer Umpumpung im Batch-Verfahren erfolgt.

EP 1 777 267 A2

## Beschreibung

Die Erfindung betrifft ein Verfahren zur Herstellung einer Metall-Verbindungen einer Azo-Verbindung unter Einsatz einer Umpumpung in einem Batch-Verfahren, die Verwendung der Metallverbindungen als Pigmente und die Verwendung der Pigmente.

Metallkomplexpigmente von Metallen mit einer Azo-Verbindung der folgenden Formel

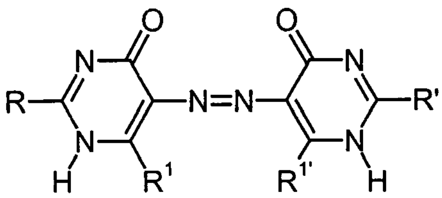

in der
R und R' unabhängig voneinander für OH, $NH_2$, NH-CN, Arylamino oder Acylamino stehen und
$R^1$ und $R^{1'}$ unabhängig voneinander -OH oder $-NH_2$ bedeuten,
sowie Einlagerungsverbindungen daraus sind in der Literatur umfangreich beschrieben, z.B.:

- DE-A-2 064 093
- US-A-4,622,391
- EP 0 994 162 A1
- EP 0 994 163 A1
- EP 0 994 164 A1
- DE 103 28 999 A1.

Es ist bekannt, dass es bei der Herstellung der oben beschriebenen Metallverbindungen bzw. Einlagerungsverbindungen daraus zu relativ starken Schwankungen der Produkteigenschaften kommt. Insbesondere unter Produktionsbedingungen im industriellen Maßstab insbesondere im Batch-Verfahren unterliegen bestimmte Parameter des erhaltenen Materials, wie z.B. die spezifische Oberfläche nach BET, mehr oder weniger starken Schwankungen. Dies ist natürlich nachteilig, da die Abnehmer der genannten Verbindungen eine konstante Produktqualität wünschen. Weiterhin werden insbesondere für die Herstellung von Farbfiltern für Flüssigkristallanzeigen Produktqualitäten mit hohen BET-Oberflächen gewünscht.

Eine gewisse Vereinheitlichung der Produktqualität (Reproduzierbarkeit) ist möglich, durch einen Temperschritt, wie er beispielsweise in der EP-A1-0994162 oder der DE 10328999 A1 beschrieben ist. Dabei nimmt man jedoch eine Verringerung der spezifischen Oberfläche nach BET in Kauf, was insbesondere für die Anwendung zur Herstellung von Farbfiltern für Flüssigkristallanzeigen nachteilig ist.

Ein Nachteil der im genannten Stand der Technik beschriebenen Batch-Verfahren ist, dass insbesondere bei größeren Kesselvolumen die Aufheizung der Mischung und die Verteilung von während des Herstellungsverfahrens zugesetzten Reaktanden und insbesondere von Säuren und Basen nicht in der gewünschten Gleichmäßigkeit erfolgen kann. Eine Folge solcher Temperatur-, Konzentrations- und pH-Gradienten ist, dass miteinander konkurrierende Reaktionen erfolgen können, was zu einer schlechten Reproduzierbarkeit bzw. schwankenden Produktqualität führt. Weiterhin erfordern die Batch-Verfahren relativ lange Reaktionszeiten, insbesondere im industriellen Maßstab. Die geschilderten Nachteile des Batch-Verfahrens werden ausführlich in der EP-A-1142960 beschrieben. Das dort beschriebene kontinuierliche Herstellungsverfahren erfordert einen erheblichen zusätzlichen apparativen Aufwand, insbesondere mehrere Eduktbehälter, einen Mischreaktor und Mengen- und pH-gesteuerte Regel-, Pump- und Sicherheits-Systeme, die extrem genau aufeinander abgestimmt sein müssen.

Aufgabe der Erfindung war es somit, ein kostengünstiges Verfahren mit hoher Raum-Zeit-Ausbeute zur Herstellung der erwähnten Metallverbindungen zu finden, dass die Reproduzierbarkeit der Herstellung der Metallverbindungen oder Einlagerungsverbindungen daraus erhöht und insbesondere die reproduzierbare Bildung eines Produktes ermöglicht, das über eine hohe BET-Oberfläche verfügt. Schwankungen in der Produktqualität sollten möglichst klein sein.

Die Erfindung betrifft ein Verfahren zur Herstellung von Metall-Verbindungen einer Azo-Verbindung der Formel (I)

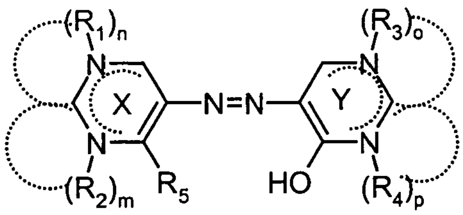

(I)

oder tautomere Strukturen davon,
worin
die mit X und Y bezeichneten Ringe je einen oder zwei Substituenten aus der Reihe =O, =S, $=NR_7$, $-NR_6R_7$, $-OR_6$, $-SR_6$, $-COOR_6$, -CN, $-CONR_6R_7$, $-SO_2R_8$, 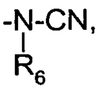

Alkyl, Cycloalkyl, Aryl, Aralkyl tragen können,
wobei die Summe der endo- und exocyclischen Doppelbindungen für jeden der Ringe X und Y drei ist,
worin

$R_6$ Wasserstoff, Alkyl, Cycloalkyl, Aryl oder Aralkyl ist,

$R_7$ Wasserstoff, Cyano, Alkyl, Cycloalkyl, Aryl, Aralkyl oder Acyl ist und

$R_8$ Alkyl, Cycloalkyl, Aryl oder Aralkyl ist,

$R_1$, $R_2$, $R_3$, $R_4$ für Wasserstoff, Alkyl, Cycloalkyl, Aryl oder Aralkyl stehen und weiterhin, wie in Formel (I) durch die unterbrochenen Linien angedeutet wird, 5- oder 6-gliedrige Ringe ausbilden können, an die weitere Ringe ankondensiert sein können,

$R_5$ -OH, $-NR_6R_7$, Alkyl, Cycloalkyl, Aryl oder Aralkyl bedeutet, worin $R_6$ und $R_7$ wie oben definiert sind,

wobei in den für $R_1$ bis $R_8$ genannten Substituenten, die CH-Gruppen enthalten, die Wasserstoffatome in den CH-Gruppen substituiert sein können,
und m, n, o, p l (eins), oder für den Fall, dass von den Ring-Stickstoffatomen, an denen die entsprechenden Substituenten $R_1$ bis $R_4$ sitzen, Doppelbindungen ausgehen, wie in Formel (I) durch die punktierten Linien angedeutet wird, auch 0 (null) bedeuten können,
und die gegebenenfalls eine Verbindung eingelagert enthalten,
dadurch gekennzeichnet, dass die Herstellung unter Verwendung einer Umpumpung in einem Batch-Verfahren erfolgt.
Der Begriff "Batch-Verfahren" meint, wie dem Fachmann gut bekannt ist, ein diskontinuierliches Verfahren. D.h., die Herstellung der Metall-Verbindungen wird nicht kontinuierlich sondern ansatz- bzw. chargenweise durchgeführt. Nach Durchführung eines Reaktionsansatzes wird das Produkt isoliert. Im Gegensatz dazu werden beim kontinuierlichen Verfahren kontinuierlich Edukte zugeführt und Produkt abgeführt.
Die "Umpumpung" bedeutet erfindungsgemäß, dass Mittel vorgesehen sind, mit denen während der Herstellung dem Reaktor Inhalt entnommen und wiederzugeführt werden kann. Eine bevorzugte Ausgestaltung einer solchen Umpumpung besteht darin, dass der verwendete Reaktor, insbesondere ein Rührkessel ein vorzugsweise außerhalb des Reaktors liegendes Rohrleitungssystem aufweist. Das Rohrleitungssystem ist mit dem Reaktor bzw. Reaktorinhalt an mindestens zwei verschiedenen Stellen verbunden. Das Rohrleitungssystem weist Mittel auf, mit denen dem Reaktor an einer oder mehreren Stellen Reaktorinhalt entnommen werden kann und nach Passieren des Rohrleitungssystems an einer oder mehreren anderen Stellen wieder zugeführt werden kann. Derartige Mittel sind insbesondere Pumpen. Das erfindungsgemäß verwendete Umpumpungssystem weist bevorzugt Dosiereinrichtungen auf, die es ermöglichen, in das außerhalb des Reaktors liegende Rohrleitungssystem Reaktionspartner, beispielsweise Edukte, Lösungen von Edukten, Säuren, Basen etc. zu geben.
Ein besonders bevorzugtes erfindungsgemäßes Verfahren besteht darin, Säuren und Basen nicht direkt in den Reaktor sondern in das Umpumpungssystem zu dosieren. Ein besonders bevorzugtes erfindungsgemäßes Verfahren besteht darin, Reaktanden, Säuren und/oder Laugen bzw. Basen so zu dosieren, dass die Dosierzeit dem 0,2 fachen

- 5 fachen eines theoretischen Gesamtumpumpcyclus, insbesondere dem 1 fachen - 2 fachen entspricht. Der theoretische Gesamtumpumpcyclus bedeutet den Zeitraum, innerhalb dessen das Volumen des Reaktorinhalts das Umpumpungssystem einmal passiert hat.

Es wird angenommen, dass die erfindungsgemäß angewendete Umpumpung einen Bereich schafft, der eine vergleichsweise hohe Strömungsgeschwindigkeit aufweist. Diese Strömungsgeschwindigkeit liegt im allgemeinen höher als die Strömungsgeschwindigkeit im Rührkesselreaktor an Stellen geringer Rührwirkung, wie z.B. im Bereich oberhalb des obersten Rührblatts. Insbesondere bei Zudosierung im Bereich des Umpumpungssystems können durch die dort herrschende hohe Strömungsgeschwindigkeit lokale Konzentrationsspitzen vermieden werden. Weiterhin wird insgesamt eine bessere Durchmischung des Reaktorinhalts sichergestellt. Überraschend wird durch das erfindungsgemäße Verfahren ein Produkt erhalten, das eine höhere spezifische Oberfläche aufweist als ein Produkt, das ohne das erfindungsgemäße Umpumpungsverfahren hergestellt wurde. Weiterhin führt die Anwendung des erfindungsgemäßen Verfahrens zu einer Verringerung der Schwankungen der Produktqualität.

Erfindungsgemäß können auch mehrere Umpumpsysteme parallel eingesetzt werden.

Bevorzugt erfolgt die erfindungsgemäße Herstellung der Metallverbindungen der Azoverbindung der Formel (I) unter Verwendung einer Umpumpung in einem Rührwerkskessel. Bevorzugt erfolgt die Herstellung in einem Eintopf-Verfahren.

Bevorzugt erfolgt die Herstellung in einem Rührwerkskessel von etwa 10 $m^3$ - 100 $m^3$ Volumen. Bevorzugt erfolgt die Rührung mit 5 U/min - 200 U/min, insbesondere mit 10 U/min - 60 U/min. Vorzugsweise enthält der Reaktor Stromstörer.

Vorzugsweise wird mit der erfindungsgemäß verwendeten Umpumpung pro Stunde ein Volumen umgepumpt, das dem 0,5 fachen - 10 fachen Reaktorvolumen, insbesondere dem 1 fachen - 5 fachen Reaktorvolumen entspricht.

Vorzugsweise erfolgt die Temperierung des Reaktorinhalts über den Mantel des Reaktors. Die Temperierung kann auch durch Temperieren des umgepumpten Materials ausserhalb des Reaktors oder durch interne bzw. externe Wärmetauscher erfolgen.

Die Anwendung des erfindungsgemäßen Verfahrens führt zu einer besseren Reproduzierbarkeit der Batch-Ansätze bzw. einer Verringerung der Schwankungen der Produktqualität. Weiterhin weisen die erfindungsgemäß hergestellten Metall-Verbindungen sehr enge Partikelgrößenverteilungen sowie sehr hohe BET-Oberflächen auf. Letzteres macht sie insbesondere für LCD-Anwendungen geeignet.

Ein besonderer Vorteil des erfindungsgemäßen Verfahrens ist die im Vergleich zu kontinuierlichen Verfahren einfache Verfahrensführung und Robustheit insbesondere im industriellen Einsatz verbunden mit deutlich reduzierten Reaktionszeiten bei verbesserter Produktqualität.

Erfindungsgemäß werden unter Metall-Verbindungen einer Azo-Verbindung der Formel (I) insbesondere Metallkomplexverbindungen der Azo-Verbindung der Formel (I) und/oder salzartige Metallverbindungen der Azo-Verbindung der Formel (I) verstanden. Die Azo-Verbindung der Formel (I) liegt in den erfindungsgemäß hergestellten Metall-Verbindungen in der Regel ein- oder mehrfach deprotoniert als Anion vor, wohingegen die Metalle als Kationen vorliegen, die salzartig bis komplexartig bzw. koordinativ (also mit kovalenten Bindungsanteilen) mit dem Anion der Azo-Verbindung der Formel (I) verbunden sind. Die Formel (I) zeigt die Azo-Verbindung in der nicht deprotonierten Form, d.h. in der freien Säureform. Die Herstellung dieser komplexartigen und/oder salzartigen Metallverbindungen beruht vorzugsweise auf der Umsetzung der sauer reagierenden Azoverbindungen der Formel (I) mit Metallverbindungen, wahlweise in Gegenwart von Basen, unter Bildung der Metall-Verbindungen einer Azo-Verbindung der Formel (I).

Die erfindungsgemäß hergestellten Metallverbindungen oder die Einlagerungsverbindungen daraus können auch als Hydrate vorliegen.

Die oben genannte Anzahl der Substituenten an den mit X und Y bezeichneten Ringen (ein oder zwei Substituenten) ist erfindungsgemäß so zu verstehen, dass die eingezeichneten Substituenten $R_1$ bis $R_5$ und -OH dabei nicht mitgezählt werden. Die genannten Substituenten an den mit X und Y bezeichneten Ringen sind somit die Substituenten, die an den nicht durch $R_1$ bis $R_5$ besetzten Positionen sitzen. Mit den Substituenten $R_1$ bis $R_5$ können somit auch mehr als zwei Substituenten an den mit X und Y bezeichneten Ringen sitzen.

In einer bevorzugten Ausführungsform des erfindungsgemäßen Verfahrens steht in der Verbindung der Formel (I) der mit X gekennzeichnete Ring für einen Ring der Formel

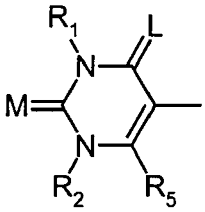 , 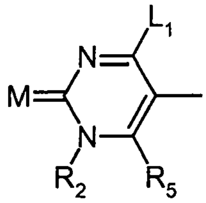 , 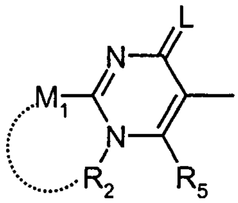

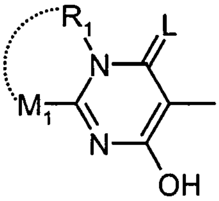 oder 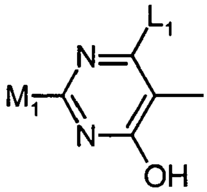

in denen

L und M unabhängig voneinander für =O, =S oder $=NR_6$ stehen,

$L_1$ Wasserstoff, $-OR_6$, $-SR_6$, $-NR_6R_7$, $-COOR_6$, $-CON-R_6R_7$, -CN, Alkyl, Cycloalkyl, Aryl, Aralkyl ist und

$M_1$ $-OR_6$, $-SR_6$, $-NR_6R_7$, $-COOR_6$, $-CONR_6R_7$, -CN, $-SO_2R_8$, 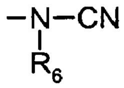 , Alkyl, Cyclo-alkyl, Aryl oder Aralkyl bezeichnen,

oder die Substituenten $M_1$ und $R_1$ oder $M_1$ und $R_2$ einen 5- oder 6-gliedrigen Ring ausbilden können, und $R_1$, $R_2$, $R_5$, $R_6$, $R_7$ und $R_8$ die oben angegebenen Bedeutungen besitzen.

Besonders bevorzugte erfindungsgemäß hergestellte Metallverbindungen sind dabei solche von Azo-Verbindungen, die in Form ihrer freien Säuren Strukturen der Formeln (II) oder (III) entsprechen

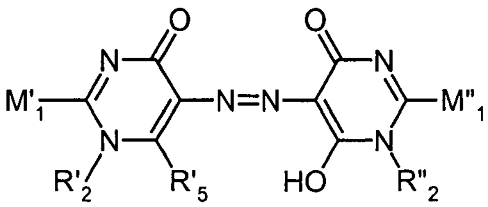 (II)

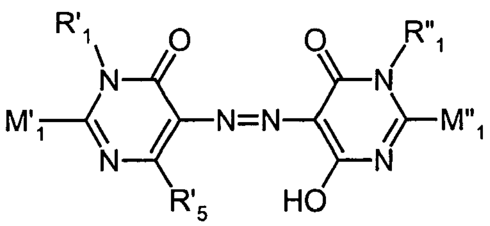 (III),

oder dazu tautomeren Formen,
in denen

| | |
|---|---|
| $R'_5$ | -OH oder $-NH_2$ bezeichnet, |
| $R'_1$, $R''_1$, $R'_2$ und $R''_2$ | jeweils für Wasserstoff stehen und |
| $M'_1$ und $M''_1$ | unabhängig voneinander für Wasserstoff, -OH, $-NH_2$, -NHCN, Arylamino oder Acylamino stehen. |

Ganz besonders bevorzugte Metallverbindungen sind dabei solche von Azo-Verbindungen der Formel (I), die in Form ihrer freien Säure einer Struktur der Formel (IV) entsprechen

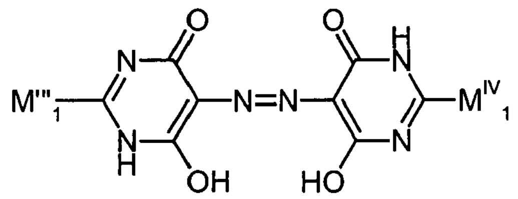

(IV),

oder tautomeren Strukturen davon,
in denen

$M'''_1$ und $M^{IV}_1$ unabhängig voneinander OH und/oder NHCN bedeuten.

Besonders bevorzugt sind Metallverbindungen von Azo-Verbindungen der Formel:

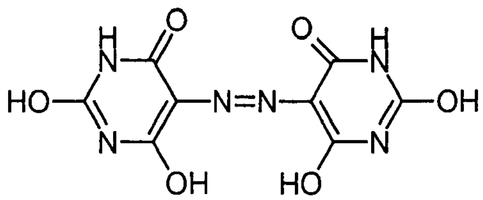

(V)

oder dazu tautomeren Strukturen.

In den vorstehenden Formeln haben die Substituenten vorzugsweise die folgenden Bedeutungen:

Substituenten in der Bedeutung von Alkyl bezeichnen vorzugsweise $C_1$-$C_6$-Alkyl, das beispielsweise durch Halogen, wie Chlor, Brom, Fluor, -OH, -CN, $-NH_2$ oder $C_1$-$C_6$-Alkoxy substituiert sein kann. Darin bedeutet $C_1$-$C_6$-Alkyl geradkettiges oder verzweigtes Alkyl mit 1 bis 6 Kohlenstoffatomen, wie Methyl, Ethyl, Propyl, Butyl, Pentyl , Hexyl einschließlich sämtlicher isomerer Formen davon.

Substituenten in der Bedeutung von Cycloalkyl bezeichnen vorzugsweise $C_3$-$C_7$-Cycloalkyl, insbesondere $C_5$-$C_6$-Cycloalkyl, das beispielsweise durch $C_1$-$C_6$-Alkyl, $C_1$-$C_6$-Alkoxy, Halogen wie Cl, Br, F, $C_1$-$C_6$-Alkoxy, -OH, -CN und $NH_2$ substituiert sein kann.

Substituenten in der Bedeutung von Aryl bezeichnen vorzugsweise Phenyl oder Naphthyl, die beispielsweise durch Halogen wie F, Cl, Br, -OH, $C_1$-$C_6$-Alkyl, $C_1$-$C_6$-Alkoxy, $-NH_2$, $-NO_2$ und -CN substituiert sein können.

Substituenten in der Bedeutung von Aralkyl bezeichnen bevorzugt Phenyl- oder Naphthyl-$C_1$-$C_4$-alkyl, die in den aromatischen Resten beispielsweise durch Halogen wie F, Cl, Br, -OH, $C_1$-$C_6$-Alkyl, $C_1$-$C_6$-Alkoxy, $-NH_2$, $-NO_2$ und -CN substituiert sein können.

Substituenten in der Bedeutung von Acyl bezeichnen vorzugsweise ($C_1$-$C_6$-Alkyl)-carbonyl, Phenylcarbonyl, $C_1$-$C_6$-Alkylsulfonyl, Phenylsulfonyl, gegebenenfalls durch $C_1$-$C_6$-Alkyl, Phenyl und Naphthyl substituiertes Carbamoyl, gegebenenfalls durch $C_1$-$C_6$-Alkyl, Phenyl und Naphthyl substituiertes Sulfamoyl oder gegebenenfalls durch $C_1$-$C_6$-Alkyl,

Phenyl oder Naphthyl substituiertes Guanyl, wobei die genannten Alkylreste beispielsweise durch Halogen wie Cl, Br, F, -OH, -CN, $-NH_2$ oder $C_1$-$C_6$-Alkoxy substituiert sein können und die genannten Phenyl- und Naphthylreste beispielsweise durch Halogen wie F, Cl, Br, -OH, $C_1$-$C_6$-Alkyl, $C_1$-$C_6$-Alkoxy, $-NH_2$, $-NO_2$ und -CN substituiert sein können.

Für den Fall, dass $M_1$ zusammen mit $R_1$ oder $M_1$ zusammen mit $R_2$ und/oder $R_1$, $R_2$, $R_3$, $R_4$, wie in den oben gezeigten Formeln durch die unterbrochenen Linien angedeutet wird, 5- oder 6-gliedrige Ringe ausbilden, handelt es sich vorzugsweise um Triazol-, Imidazol- oder Benzimidazol-, Pyrimidin- oder Chinazolin-Ringsysteme.

Als Metallverbindungen, worunter wie bereits dargelegt, salz- oder komplexartige Metallverbindungen verstanden werden, der Azoverbindungen der Formeln (I) bis (V) kommen vorzugsweise die Salze und Komplexe der Mono-, Di-, Tri- und Tetraanionen der Azoverbindungen der Formeln (I) bis (V) in Betracht. Geeignete Metalle werden zweckmäßig ausgewählt aus einem oder mehreren Metallen, die aus der Gruppe ausgewählt werden, die besteht aus: Li, Na, K, Mg, Ca, Sr, Ba, Al, Sn, Pb, Sc, Ti, V, Cr, Mn, Fe, Co, Ni, Cu, Zn, Y, Zr, Nb, Mo, Cd, Hf, Ta, W, La, Ce, Pr und Nd.

Besonders bevorzugt sind Salze und Komplexe der Formeln (I) bis (V) mit zwei- oder dreiwertigen Metallen, ganz besonders die Nickelsalze und -komplexe. In einer bevorzugten Ausführunsgform des erfindungsgemäßen Verfahrens wird ein Ni-Salz bzw. eine Ni-Komplexverbindung der Azoverbindung der Formel (I) hergestellt.

Bevorzugt handelt es sich bei der Metallverbindung um den Azobarbitursäure-Nickel-1:1-Komplexes der Struktur

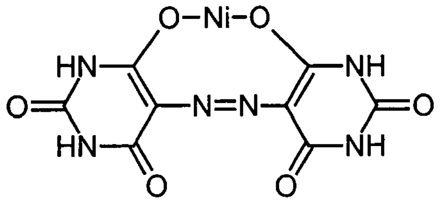

(VI)

oder eine tautomere Struktur dazu.

Die erfindungsgemäß hergestellten Metallverbindungen können gegebenenfalls eine oder mehrere Verbindungen eingelagert enthalten. Bevorzugt handelt es sich bei der eingelagerten Verbindung um eine organische Verbindung, d.h. um eine solche Verbindung die mindestens ein kovalent gebundenes Kohlenstoffatom aufweist. Bei den erfindungsgemäß hergestellten Zusammensetzungen aus Metallverbindung und eingelagerter organischer Verbindung kann es sich um Einschlussverbindungen, Interkalationsverbindungen oder feste Lösungen handeln.

Bevorzugt handelt es sich um Einschlußverbindungen, Interkalationsverbindungen bzw. feste Lösungen eines Azobarbitursäure-Nickel-1:1-Komplexes der Struktur

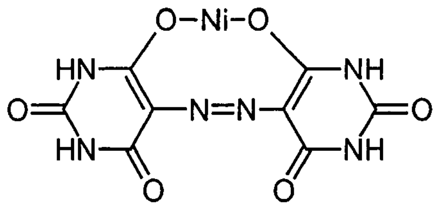

(VI)

oder einer tautomeren Struktur dazu und mindestens einer anderen darin eingeschlossenen organischen Verbindung.

Besonders bevorzugt handelt es sich um Interkalationsverbindungen der vorstehend beschriebenen Metallverbindung der Formel (VI) mit Melamin in einem molaren Verhältnis von 1 : 2.

Im allgemeinen bilden die erfindungsgemäß hergestellten Metallverbindungen schichtförmigen Kristallgitter, bei dem die Bindung innerhalb einer Schicht im wesentlichen über Wasserstoffbrücken und/oder Metallionen erfolgt. Vorzugsweise handelt es sich dabei um Metallkomplexe, die ein Kristallgitter ausbilden, das aus im wesentlichen ebenen Schichten besteht.

In einer bevorzugten Ausführungsform des erfindungsgemäßen Verfahrens erfolgt die erfindungsgemäße Herstellung der Metallkomplexe der Azoverbindung der Formel (I) bzw. der Einlagerungsverbindungen davon in Gegenwart von Impfkristallen. Bevorzugt besitzen die Impfkristalle die gleiche chemische Struktur wie die nach dem erfindungsgemäßen Verfahren herzustellenden Metallverbindungen der Azoverbindung der Formel (I) bzw. die Einlagerungsverbindungen davon. Insbesondere werden, wenn das herzustellende Produkt eine Zusammensetzung aus einer Metallverbindung der Azoverbindung der Formel (I) und einer darin eingelagerten Verbindung ist, auch Impfkristalle einer solchen Einschlusszusammensetzung verwendet. Es hat sich dabei überraschend herausgestellt, dass die physikalischen Eigenschaften der verwendeten Impfkristalle nicht notwendigerweise die physikalischen Eigenschaften der herzustellenden Metallverbindungen bestimmen. So werden beispielsweise Metallverbindungen mit hoher spezifischer Oberfläche nach BET auch dann erhalten, wenn die eingesetzten Impfkristalle eine vergleichweise geringe spezifische Oberfläche nach BET aufweisen.

Bevorzugt erfolgt die erfindungsgemäße Herstellung in Gegenwart von 1 ppm - 10.000 ppm an Impfkristallen bezogen auf die theoretisch erhältliche Menge der herzustellenden Metallverbindung eines gegebenen Reaktionsansatzes, insbesondere von 10 ppm - 5.000 ppm, ganz besonders bevorzugt von 50 ppm - 3.000 ppm, insbesondere von 100 ppm - 2.000 ppm.

Erfindungsgemäß können vorteilhaft Metallverbindungen der Azoverbindungen der Formel (I), die vorzugsweise eine Verbindung eingelagert enthalten, bzw. ihnen entsprechende Pigmente mit sehr hohen spezifischen Oberflächen nach BET, insbesondere für LCD-Anwendungen hergestellt werden.

So können nach dem erfindungsgemäßen Verfahren spezifische Oberflächen nach BET der Metallverbindungen der Azoverbindungen der Formel (I) bzw. der Zusammensetzung mit mindestens einer eingelagerten Verbindung davon mit mindestens 160 $m^2/g$, bevorzugt von mindestens 165 $m^2/g$ erhalten werden. Insbesondere durch Zusatz von Impfkristallen können insbesondere spezifische Oberflächen von mehr als 180 $m^2/g$, wie zwischen 180 $m^2/g$ und 240 $m^2/g$, insbesondere zwischen 180 $m^2/g$ und 210 $m^2/g$ erhalten werden. Die spezifische Oberfläche wird nach DIN 66131 ermittelt: Bestimmung der spezifischen Oberfläche von Feststoffen durch Gasadsorption nach Brunauer, Emmett und Teller (B.E.T.).

Für bestimmte Anwendungen kann es sinnvoll sein, die nach dem erfindungsgemäßen Verfahren erhaltenen Metallverbindungen bzw. Einlagerungsverbindungen davon zu tempern, wie z.B. in der EP-A1-0994162 beschrieben. Durch das Tempern wird im allgemeinen eine engere Partikelgrößenverteilung erhalten. Wie eingangs beschrieben, geht damit im allgemeinen aber auch eine Verringerung der spezifischen Oberfläche einher. Dabei wird in einer bevorzugten Ausführungsform die hergestellte wässrige Suspension der Metallverbindung bzw. der Einlagerungsverbindung davon in wenigstens zwei pH-Stufen getempert. Dadurch kann gegenüber einer einstufigen Temperung eine deutlich verbesserte Farbstärke erhalten werden. Zweckmäßig erfolgt die mehrstufige Temperung in jeder Temperstufe bei Temperaturen zwischen 80 bis 125°C. Bevorzugt wird die mehrstufige Temperung in Gegenwart von Wasser und gegebenenfalls organischen Lösungsmitteln bei pH-Werten im Bereich von 0 bis 4 durchgeführt. Vorzugsweise liegt der pH-Wert von wenigstens einer Temperungsstufe zwischen 2 und 4, insbesondere zwischen 2,5 und 3,5. Vorzugsweise liegt der pH-Wert von einer zweiten Temperungsstufe zwischen 0 und 3, besonders bevorzugt zwischen 1 und 2,5. Vorzugsweise unterscheiden sich die pH-Werte von zwei Temperungsstufen um 0,5 bis 3 Einheiten, vorzugsweise um 1 bis 2 Einheiten. Vorzugsweise dauern wenigstens zwei Temperungsstufen unabhängig voneinander zwischen 0,25h und 24h, insbesondere zwischen 1h und 12h, ganz besonders bevorzugt zwischen 2h und 8h.

Die Umpumpung sowie gegebenenfalls die Verwendung der Impfkristalle ist aber auch bei Anwendung eines Temperverfahrens dennoch sinnvoll, da der Vorteil der geringeren Schwankung der Produktqualität bestehen bleibt und vor der Tempererung von einem höheren Niveau der spezifischen Oberfläche ausgegangen werden kann.

Die Verwendung der Umpumpung sowie gegebenenfalls der Impfkristalle führt nicht notwendigerweise zu spezifischen Oberflächen nach BET von mehr als 180 $m^2/g$. Der Fachmann weiß nämlich, dass die Einstellung einer spezifischen Oberfläche auch von anderen Herstellparametern abhängt, wie z.B. der Herstellungstemperatur. Das erfindungsgemäße Verfahren führt jedoch unter sonst gleichen Herstellbedingungen mit höherer Reproduzierbarkeit zu einer höheren spezifischen Oberfläche nach BET der erfindungsgemäß hergestellten Metallverbindungen.

Im Rahmen dieser Anmeldung werden die Metallverbindungen der Azoverbindungen der Formel (I), die vorzugsweise wenigstens eine Verbindung eingelagert enthalten und unter Anwendung der Umpumpung hergestellt wurden, auch als erfindungsgemäß hergestellte Pigmente bezeichnet.

Als Metallverbindungen kommen auch solche in Frage, bei denen eine metallhaltige Verbindung, z.B. ein Salz oder ein Metallkomplex in das Kristallgitter eines anderen Metallkomplexes z.B. des Nickelkomplexes eingebaut ist. In diesem Fall kann beispielsweise in Formel (VI) ein Teil des Metalls, z.B. des Nickels durch andere Metallionen ersetzt sein oder es können weitere Metallionen in eine mehr oder weniger starke Wechselwirkung mit der Metallverbindung, vorzugsweise einem Nickelkomplex treten.

Einschlussverbindungen, Interkalationsverbindungen und feste Lösungen von Metallkomplexen an sich sind aus der Literatur bekannt. Sie werden ebenso wie deren Herstellung beispielsweise in der EP 0 074 515, der EP 0 073 463 , der EP 0994163 sowie der EP 0994162 (dort Seite 5, Zeile 40 bis Seite 7, Zeile 58) beschrieben. Insoweit kann vollinhaltlich auf die Aufzählung der in Frage kommenden Verbindungen in diesen Druckschriften Bezug genommen werden.

Besonders bevorzugt werden als eingelagerte Verbindungen Melamin oder Melaminderivate, insbesondere solche der Formel (VII)

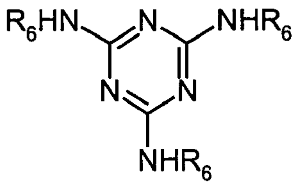

(VII)

eingesetzt, worin

$R_6$ für Wasserstoff oder $C_1$-$C_4$-Alkyl, das gegebenenfalls mit OH-Gruppen substituiert ist, steht,

ganz besonders bevorzugt, worin

$R_6$ für Wasserstoff steht.

Die Menge an eingelagerter Substanz, die in das Kristallgitter des Metallkomplexes eingelagert werden kann, liegt in der Regel bei 5 % bis 200 Gew.-%, bezogen auf die Menge an Metallverbindung. Bevorzugt eingelagert werden 10 bis 100 Gew.-%. Es handelt sich hierbei um die Menge an eingelagerter Substanz, die durch geeignete Lösungsmittel nicht auswaschbar ist und die sich aus der Elementaranalyse ergibt. Naturgemäß kann auch mehr oder weniger als die genannte Menge an Substanz zugesetzt werden, wobei man gegebenenfalls darauf verzichten kann, einen Überschuß auszuwaschen. Bevorzugt sind Mengen von 10 bis 150 Gew.-% bezogen auf die Menge an Metallverbindung.

Die Herstellung der Metallverbindungen bzw. der Einlagerungsverbindungen davon erfolgt beispielsweise wie in EP 0 074 515, EP 0 073 463, EP 0994163 sowie der EP 0994162 beschrieben. Nach der Synthese der Azoverbindung wird im allgemeinen in Gegenwart der zu interkalierenden Verbindung mit einem Metallsalz komplexiert. Im Falle der technisch interessanten Interkalationsverbindungen der Metallkomplexe der zwei- und dreiwertigen Metalle, besonders der technisch und wirtschaftlichen wichtigen Interkalationsverbindung des Azobarbitursäure-Nickel-Komplexes, erfolgen Komplexierung und Interkalation sowie auch die nachfolgende Isolierung zweckmäßig im sauren pH-Bereich.

Als Metallsalz kommen vorzugsweise wasserlösliche Metallsalze der oben genannten Metalle in Frage, insbesondere Chloride, Bromide, Acetate, Nitrate usw. bevorzugt des Nickels in Frage. Bevorzugt eingesetzte Metallsalze besitzen eine Wasserlöslichkeit von mehr als 20 g/l, insbesondere mehr als 50 g/l bei 20 °C.

Man kann auch Mischungen dieser Salze, die verschiedene der genannten Metalle enthalten können, verwenden. Die Verwendung von solchen Salzmischungen empfiehlt sich insbesondere für die Erzielung von Zwischentönen der farbigen Endprodukte.

Die bei der Herstellung erhaltene Suspension wird vorzugsweise filtriert und der so erhaltene Presskuchen kann gegebenenfalls nach Waschen mit Wasser getrocknet werden.

Dabei kommen einerseits übliche Trocknungsverfahren wie die Schaufeltrocknung usw. in Frage. Mit derartigen Trocknungsverfahren und anschließender Mahlung des Pigments auf übliche Weise werden pulverförmige Pigmente erhalten.

Bevorzugt wird der Presskuchen als wäßrige Slurry sprühgetrocknet. Der zu versprühende Slurry besitzt vorzugsweise einen Feststoffanteil von 10 bis 40 Gew.-%, insbesondere 15 bis 30 Gew.-%.

Die Erfindung betrifft weiterhin ein Verfahren zur Herstellung von Pigmentpräparationen, worin wenigstens eine erfindungsgemäß hergestellte Metallverbindung oder eine Einlagerungsverbindung davon und wenigstens ein Dispergiermittel vermischt werden. Diese Pigmentpräparationen dienen vorzugsweise der Einarbeitung in wässrige Systeme.

Bezüglich geeigneter Dispergiermittel kann auf den eingangs erwähnten Stand der Technik verwiesen werden, insbesondere die EP-A1-0994164, Seite 8, Zeile 56 Seite 11, Zeile 23, deren Offenbarung zum Gegenstand dieser Anmeldung gehören. Besonders bevorzugt enthält die Pigment-Präparation mehr als 90 insbesondere mehr als 95, vorzugsweise mehr als 97 Gew.-% an Pigment (erfindungsgemäß hergestellte Metallverbindung + gegebenenfalls darin eingelagerte Verbindung(en)) und Dispergiermittel.

Die Erfindung betrifft bevorzugt die Verwendung der erfindungsgemäß hergestellten Metallverbindungen oder der Einlagerungsverbindungen davon als Pigment für Farbfilter in Flüssigkristallanzeigen.

Die Erfindung betrifft weiterhin ein Verfahren zur Herstellung von Farbfiltern in Flüssigkristallanzeigen, das die Verwendung der erfindungsgemäß hergestellten Metallverbindungen oder der Einlagerungsverbindungen davon vorzugsweise jeweils mit einer spezifischen Oberfläche nach BET von mindestens 160 $m^2/g$ umfasst.

Die Erfindung betrifft weiterhin die Verwendung der erfindungsgemäß hergestellten Metallverbindungen oder

der Einlagerungsverbindungen davon zur Herstellung eines Photolacks, der wenigstens ein photohärtbares Monomer und wenigstens einen Photoinitiator enthält. Die Erfindung betrifft weiterhin die Verwendung der erfindungsgemäß hergestellten Metallverbindungen oder der Einlagerungsverbindungen davon zur Herstellung eines Farbfilter und daraus hergestellten Flüssigkristallanzeigen. Bei der Herstellung der Farbfilter für Flüssigkristallanzeigen werden die erfindungsgemäß hergestellten Metallverbindungen oder die Einlagerungsverbindungen davon (bevorzugt mit Melamin) in einem organischen Lösungsmittel gegebenenfalls unter Zusatz eines Bindemittelharzes und/oder Dispergiermittels gemahlen, anschließend unter Zusatz von photohärtbaren Monomeren, Photoreaktionsstarter und ggf. weiterem Bindemittel und/oder Lösungsmittel zu einem Photolack verarbeitet, der im Anschluss daran mittels geeigneter Beschichtungsverfahren wie z.B. Roller-, Spray-, Spin-, Dip- oder Air-Knife-Coating auf ein geeignetes Substrat, im Allgemeinen eine Glasplatte aufgetragen wird, mittels Photomaske belichtet und anschließend gehärtet und zum fertigen farbigen Filter entwickelt wird.

Die erfindungsgemäß hergestellen Metallverbindungen oder Einlagerungsverbindungen davon oder Pigmentpräparationen eignen sich überdies hervorragend für alle Pigmentanwendungszwecke.

Zum Beispiel eignen sie sich zum Pigmentieren von Lacken aller Art für die Herstellung von Druckfarben, Leimfarben oder Binderfarben, für die Massefärbung von synthetischen, halbsynthetischen oder natürlichen makromolekularen Stoffen, wie z.B. Polyvinylchlorid, Polystyrol, Polyamid, Polyethylen oder Polypropylen. Sie können auch für die Spinnfärbung von natürlichen, regenerierten oder künstlichen Fasern, wie z.B. Cellulose-, Polyester-, Polycarbonat-, Polyacrylniril- oder Polyamidfasern, sowie zum Bedrucken von Textilien und Papier verwendet werden. Aus diesen Pigmenten können feinteilige, stabile, wässrige Pigmentierungen von Dispersions- und Anstrichfarben, die für die Papierfärbung, für den Pigmentdruck von Textilien, für den Laminatdruck oder für die Spinnfärbung von Viskose brauchbar sind, durch Mahlen oder Kneten in Gegenwart von nichtionogenen, anionischen oder kationischen Tensiden hergestellt werden. Die nach dem erfindungsgemäßen Verfahren hergestellten Pigmente eignen sich hervorragend für Ink Jet Anwendungen und aufgrund ihrer vergleichsweise hohen spezifischen Oberfläche nach BET für Farbfilter für Flüssigkristallanzeigen.

## Beispiele

### Beispiel 1 Beispiel 1 ist nicht erfindungsgemäß.

In einem 20m$^3$ Reaktor mit Mantelheiz-/kühl-System, Rührer und Stromstörer werden 6.000 Liter 80°C heißes Wasser bei einer Rührgeschwindigkeit von 20 U/min vorgelegt. 380 kg wasserfeuchte Paste von Diazobarbitursäure mit einem Trockengehalt von 81 %, entsprechend 308 kg trocken, werden eingetragen.

Die Temperatur wird auf 80°C gehalten und bei dieser Temperatur 268 kg Barbitursäure eingetragen. Nach 2 Stunden wird mit 30%iger Kaliumhydroxidlösung innerhalb 2 Stunden auf pH 5,0 gestellt, wobei die Kaliumhydroxidlösung auf die Oberfläche in den Reaktor dosiert wird. Anschließend wird 3 Stunden bei pH 5,0 und 80°C nachgerührt. Danach wird mit Wasser auf 15.000 Liter verdünnt. Anschließend wird auf 90°C erwärmt und bei dieser Temperatur 500 kg Melamin eingetragen. Danach werden 1.150 kg 22,5%iger Nickelchloridlösung auf die Oberfläche in den Reaktor innerhalb 1 Stunde eindosiert. Zwei Stunden wird nachgerührt, um eine möglichst vollständige Umsetzung zu erreichen. Dann wird mit 30%iger Kaliumhydroxidlösung innerhalb 1 Stunde auf pH 5,0 gestellt, wobei die Kaliumhydroxidlösung auf die Oberfläche in den Reaktor eindosiert wird.

Danach wird über eine Dosierung von Salzsäure auf die Oberfläche in den Reaktor innerhalb 1 Stunde auf pH 2,5 gestellt. Die Temperatur wird auf 98°C erhöht und 4 Stunden getempert. Danach wird mit 30%iger Kaliumhydroxidlösung innerhalb 2 Stunden auf pH 5,0 gestellt, wobei die Kaliumhydroxidlösung auf die Oberfläche in den Reaktor eindosiert und die Temperatur auf 80°C geregelt wird.

Der Reaktor läßt sich bis auf geringe Anbackungen vollständig entleeren. Die Pigment-Slurry wird auf einer Filterpresse isoliert, elektrolytfrei gewaschen und bei 80°C getrocknet. Man erhält ein Produkt mit einer BET-Oberfläche von 149 m$^2$/g.

### Beispiel 2 Beispiel 2 ist erfindungsgemäß.

In einem 20m$^3$ Reaktor mit Mantelheiz-/kühl-System, Rührer, Stromstörer und Umpumpsystem werden 6.000 Liter 80°C heißes Wasser bei einer Rührgeschwindigkeit von 20 U/min vorgelegt. 380 kg wasserfeuchte Paste von Diazobarbitursäure mit einem Trockengehalt von 81 %, entsprechend 308 kg trocken, werden eingetragen.

Die Temperatur wird auf 80°C gehalten und bei dieser Temperatur 268 kg Barbitursäure eingetragen. Die Umpumpung wird eingeschaltet und auf 15m$^3$/h gestellt. Nach 1 Stunde Umpumpung wird mit 30%iger Kaliumhydroxidlösung innerhalb 1 Stunde auf pH 5,0 gestellt, wobei die Kaliumhydroxidlösung auf die Oberfläche in den Reaktor dosiert wird. Anschließend wird 2 Stunden bei pH 5,0 und 80°C unter Umpumpen nachgerührt. Danach wird mit Wasser auf 15.000 Liter verdünnt. Anschließend wird auf 90°C erwärmt und bei dieser Temperatur 500 kg Melamin eingetragen.

Die Umpumpung wird auf $30 m^3/h$ gestellt. Danach werden 1.150 kg 22,5%iger Nickelchloridlösung auf die Oberfläche in den Reaktor innerhalb 1 Stunde eindosiert. 90 Minuten wird unter Umpumpen nachgerührt, um eine möglichst vollständige Umsetzung zu erreichen. Dann wird mit 30%iger Kaliumhydroxidlösung innerhalb 1 Stunde auf pH 5,0 gestellt, wobei die Kaliumhydroxidlösung auf die Oberfläche in den Reaktor eindosiert wird.

Danach wird über eine Dosierung von Salzsäure auf die Oberfläche in den Reaktor innerhalb 1 Stunde auf pH 2,5 gestellt. Die Temperatur wird auf 98°C erhöht und 4 Stunden getempert. Danach wird mit 30%iger Kaliumhydroxidlösung innerhalb 1 Stunde auf pH 5,0 gestellt, wobei die Kaliumhydroxidlösung auf die Oberfläche in den Reaktor eindosiert und die Temperatur auf 80°C geregelt wird.

Der Reaktor läßt sich praktisch ohne Anbackungen vollständig entleeren. Die Pigment-Slurry wird auf einer Filterpresse isoliert, elektrolytfrei gewaschen und bei 80°C getrocknet. Man erhält ein weitgehend einheitliches Produkt mit enger Partikelgrößenverteilung und einer BET-Oberfläche von $162 m^2/g$.

<u>Beispiel 3</u> **Beispiel 3 ist erfindungsgemäß.**

In einem $20 m^3$ Reaktor mit Mantelheiz-/kühl-System, Rührer, Stromstörer und Umpumpsystem werden 6.000 Liter 80°C heißes Wasser bei einer Rührgeschwindigkeit von 20 U/min vorgelegt. 380 kg wasserfeuchte Paste von Diazobarbitursäure mit einem Trockengehalt von 81 %, entsprechend 308 kg trocken, werden eingetragen.

Die Temperatur wird auf 80°C gehalten und bei dieser Temperatur 268 kg Barbitursäure eingetragen. Die Umpumpung wird eingeschaltet und auf $15 m^3/h$ gestellt. Nach 1 Stunde Umpumpung wird mit 30%iger Kaliumhydroxidlösung innerhalb 30min auf pH 5,0 gestellt, wobei die Kaliumhydroxidlösung in die Umpumpung dosiert wird. Anschließend wird 2 Stunden bei pH 5,0 und 80°C unter Umpumpen nachgerührt. Danach wird mit Wasser auf 15.000 Liter verdünnt. Anschließend wird auf 90°C erwärmt und bei dieser Temperatur 500 kg Melamin eingetragen. Die Umpumpung wird auf $30 m^3/h$ gestellt. Danach werden 1.150 kg 22,5%iger Nickelchloridlösung über den Umpumpkreislauf innerhalb 30min eindosiert. 90 Minuten wird unter Umpumpen nachgerührt, um eine möglichst vollständige Umsetzung zu erreichen. Dann wird mit 30%iger Kaliumhydroxidlösung innerhalb 30min auf pH 5,0 gestellt, wobei die Kaliumhydroxidlösung in die Umpumpung eindosiert wird.

Danach wird über eine Dosierung von Salzsäure in den Umpumpkreislauf innerhalb 30 min auf pH 2,5 gestellt. Die Temperatur wird auf 98°C erhöht und 4 Stunden getempert. Danach wird mit 30%iger Kaliumhydroxidlösung innerhalb 30min auf pH 5,0 gestellt, wobei die Kaliumhydroxidlösung in den Umpumpkreislauf eindosiert und die Temperatur auf 80°C geregelt wird.

Der anbackungsfreie Reaktor läßt sich sehr leicht praktisch vollständig entleeren. Die homogene Pigment-Slurry wird auf einer Filterpresse isoliert, elektrolytfrei gewaschen und bei 80°C getrocknet. Man erhält ein sehr einheitliches Produkt mit sehr enger Partikelgrößenverteilung und einer BET-Oberfläche von $167 m^2/g$.

<u>Beisniel 4</u> **Beispiel 4 ist erfindungsgemäß.**

Man lässt in dem Ansatz von Beispiel 3 etwa 20 Liter der Produktsuspension als Impfkristalle im Reaktor zurück und arbeitet dann erneut wie in Beispiel 3 beschrieben. Man erhält ein sehr einheitliches Produkt mit sehr enger Partikelgrößenverteilung und einer BET-Oberfläche von $182 m^2/g$.

Die Beispiele zeigen, dass nach dem Verfahren der Erfindung Produkte mit vergleichsweise hoher BET-Oberfläche mit relativ geringen Schwankungen erhalten werden. Weiterhin zeigen die erfindungsgemäßen Beispiele, dass die Reaktionszeiten bei Einsatz der Umpumpung deutlich verringert werden können. Beispiel 4 zeigt, dass die Verwendung von Impfkristallen eine weitere Erhöhung der spezifischen Oberfläche nach BET bewirkt.

**Patentansprüche**

1. Verfahren zur Herstellung von Metall-Verbindungen einer Azo-Verbindung der Formel (I)

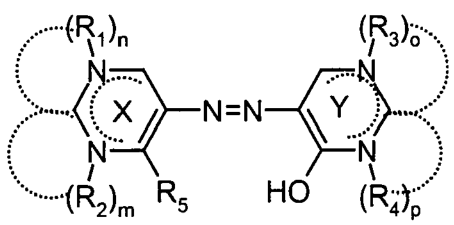

(I)

oder tautomere Strukturen davon,
worin die mit

X und Y bezeichneten Ringe je einen oder zwei Substituenten aus der Reihe

=O, =S, $=NR_7$, $-NR_6R_7$, $-OR_6$, $-SR_6$, $-COOR_6$, -CN, $-CONR_6R_7$, $-SO_2R_8$, 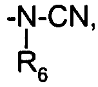

Alkyl, Cycloalkyl, Aryl, Aralkyl tragen können,
wobei die Summe der endo- und exocyclischen Doppelbindungen für jeden der Ringe X und Y drei ist,
worin

$R_6$ Wasserstoff, Alkyl, Cycloalkyl, Aryl oder Aralkyl ist,
$R_7$ Wasserstoff, Cyano, Alkyl, Cycloalkyl, Aryl, Aralkyl oder Acyl ist und
$R_8$ Alkyl, Cycloalkyl, Aryl oder Aralkyl ist,

$R_1$, $R_2$, $R_3$, $R_4$ für Wasserstoff, Alkyl, Cycloalkyl, Aryl oder Aralkyl stehen und weiterhin, wie in Formel (I) durch die unterbrochenen Linien angedeutet wird, 5- oder 6-gliedrige Ringe ausbilden können, an die weitere Ringe ankondensiert sein können,
$R_5$ -OH, $-NR_6R_7$, Alkyl, Cycloalkyl, Aryl oder Aralkyl bedeutet,

worin $R_6$ und $R_7$ wie oben definiert sind,
wobei in den für $R_1$ bis $R_8$ genannten Substituenten, die CH-Gruppen enthalten, die Wasserstoffatome in den CH-Gruppen substituiert sein können,
und m, n, o, p 1 (eins), oder für den Fall, dass von den Ring-Stickstoffatomen, an denen die entsprechenden Substituenten $R_1$ bis $R_4$ sitzen, Doppelbindungen ausgehen, wie in Formel (I) durch die punktierten Linien angedeutet wird, auch 0 (null) bedeuten können,
und die gegebenenfalls eine Verbindung eingelagert enthalten,
**dadurch gekennzeichnet, dass** die Herstellung unter Einsatz einer Umpumpung in einem Batch-Verfahren erfolgt.

2. Verfahren gemäß Anspruch 1, **dadurch gekennzeichnet, dass** in der Verbindung der Formel (I) der mit X gekennzeichnete Ring für einen Ring der Formel

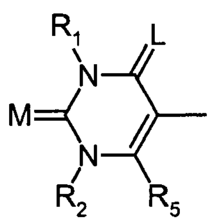 , 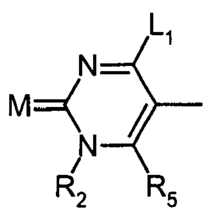 , 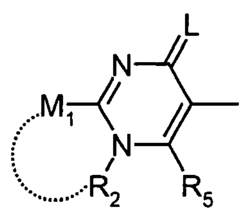

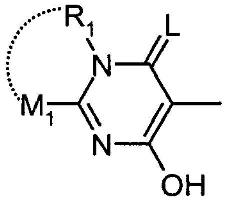 oder 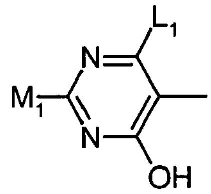

steht,

in denen

L und M unabhängig voneinander für =O, =S oder $=NR_6$ stehen,
$L_1$ Wasserstoff, $-OR_6$, $-SR_6$, $-NR_6R_7$, $-COOR_6$, $-CONR_6R_7$, -CN, Alkyl, Cycloalkyl, Aryl, Aralkyl ist und

$M_1$ $-OR_6$, $-SR_6$, $-NR_6R_7$, $-COOR_6$, $-CONR_6R_7$, -CN, $-SO_2R_8$, 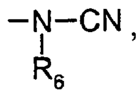 , Alkyl,

Cycloalkyl, Aryl oder Aralkyl bezeichnen,

oder die Substituenten $M_1$ und $R_1$ oder $M_1$ und $R_2$ einen 5- oder 6-gliedrigen Ring ausbilden können, und $R_1$, $R_2$, $R_5$, $R_6$, $R_7$ und $R_8$ die in Anspruch 1 angegebenen Bedeutungen besitzen.

3. Verfahren gemäß Anspruch 1, **dadurch gekennzeichnet, dass** die Azoverbindung der Formel (I) in Form ihrer freien Säure der Formel (II) oder (III) oder einer dazu tautomeren Form entspricht

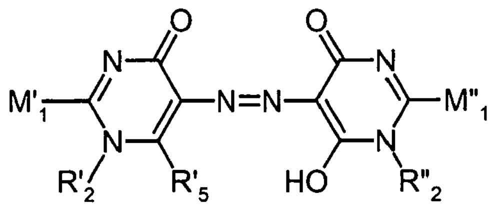

(II)

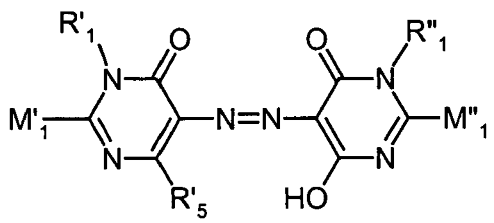

(III)

in denen

$R'_5$ -OH oder $-NH_2$ bezeichnet,
$R'_1$, $R''_1$, $R'_2$ und $R''_2$ jeweils für Wasserstoff stehen und
$M'_1$ und $M''_1$ unabhängig voneinander für Wasserstoff, -OH, $-NH_2$, -NHCN, Arylamino oder Acylamino stehen.

4. Verfahren gemäß Anspruch 1, **dadurch gekennzeichnet, dass** die Azoverbindung der Formel (I) in Form ihrer freien Säure der Formel (V) oder einer tautomeren Form davon entspricht

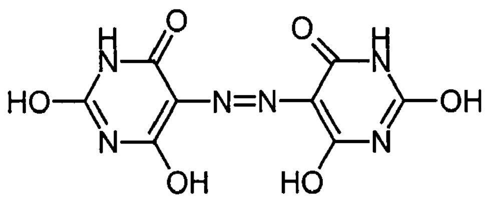

(V).

5. Verfahren gemäß einem der Ansprüche 1 bis 4, **dadurch gekennzeichnet, dass** die Metallverbindungen der Azoverbindung der Formel (I) Salze bzw. Komplexverbindungen der Mono-, Di-, Tri- und Tetraanionen der Azoverbindung der Formel (I) mit einem oder mehreren Metallen sind, die aus der Gruppe ausgewählt werden, die besteht aus: Li, Na, K, Mg, Ca, Sr, Ba, Al, Sn, Pb, Sc, Ti, V, Cr, Mn, Fe, Co, Ni, Cu, Zn, Y, Zr, Nb, Mo, Cd, Hf, Ta, W, La, Ce, Pr und Nd.

**6.** Verfahren gemäß einem der Ansprüche 1 bis 5, **dadurch gekennzeichnet, dass** als Metallverbindung ein Ni-Salz bzw. ein Ni-Komplex der Azoverbindung der Formel (I) eingesetzt wird.

**7.** Verfahren zur Herstellung von Metallverbindungen gemäß einem der Ansprüche 1 bis 6, **dadurch gekennzeichnet, dass** sie eine cyclische oder acyclische organische. Verbindung, insbesondere Melamin eingelagert enthalten.

**8.** Verfahren zur Herstellung der Metallverbindungen gemäß einem der Ansprüche 1 bis 7 oder einer Einlagerungsverbindung davon, **dadurch gekennzeichnet, dass** die Herstellung in einem Rührwerkskessel von 10 $m^3$ - 100 $m^3$ Volumen erfolgt und vorzugsweise ein Rührer mit 5 U/min - 200 U/min zugeschaltet ist.

**9.** Verfahren zur Herstellung der Metallverbindungen gemäß einem der Ansprüche 1 bis 8 oder einer Einlagerungsverbindung davon, **dadurch gekennzeichnet, dass** die Umpumpung so dimensioniert ist, dass pro Stunde ein Volumen umgepumpt wird, das dem 0,5 fachen - 10 fachen Reaktorvolumen, insbesondere dem 1 fachen - 5 fachen Reaktorvolumen entspricht.

**10.** Verfahren zur Herstellung der Metallverbindungen gemäß einem der Ansprüche 1 bis 9 oder einer Einlagerungsverbindung davon, **dadurch gekennzeichnet, dass** während der Herstellung wenigstens ein Reaktionspartner und/oder eine Lösung oder Dispersion davon und/oder eine Lauge und/oder eine Säure in das Umpumpungssystem dosiert werden.

**11.** Verfahren zur Herstellung der Metallverbindungen gemäß einem der Ansprüche 1 bis 10 oder einer Einlagerungsverbindung davon, **dadurch gekennzeichnet, dass** die Dosierzeit von wenigstens einem Reaktionspartner und/ oder Lauge und/oder Säure so abgestimmt wird, dass die Dosierzeit dem 0,2 fachen - 5 fachen eines theoretischen Gesamtumpumpcyclus entspricht.

**12.** Verfahren zur Herstellung der Metallverbindungen gemäß einem der Ansprüche 1 bis 11 oder einer Einlagerungsverbindung davon, **dadurch gekennzeichnet, dass** die Herstellung in Gegenwart von Impfkristallen erfolgt.

**13.** Verfahren zur Herstellung der Metallverbindungen gemäß einem der Ansprüche 1 bis 12 oder einer Einlagerungsverbindung davon, **dadurch gekennzeichnet, dass** eine spezifische Oberfläche nach BET des Pigmentes von mindestens 160 $m^2/g$ erhalten wird.

**14.** Verfahren zur Herstellung der Metallverbindungen gemäß einem der Ansprüche 1 bis 13 oder der Einlagerungsverbindungen davon, **dadurch gekennzeichnet, dass** die nach dem Verfahren gemäß einem der Ansprüche 1 bis 13 hergestellten Metallverbindungen oder die Einlagerungsverbindungen davon als wäßrige Slurry sprühgetrocknet werden.

**15.** Verwendung der Metallverbindungen, hergestellt gemäß einem der Ansprüche 1 bis 14, oder der Einlagerungsverbindungen davon zur Herstellung von Druckfarben, Leimfarben oder Binderfarben, für die Massefärbung von synthetischen, halbsynthetischen oder natürlichen makromolekularen Stoffen, insbesondere Polyvinylchlorid, Polystyrol, Polyamid, Polyethylen oder Polypropylen, sowie für die Spinnfärbung von natürlichen, regenerierten oder künstlichen Fasern, wie z.B. Cellulose-, Polyester-, Polycarbonat-, Polyacrylnitril- oder Polyamidfasern, sowie zum Bedrucken von Textilien und Papier.

**16.** Verwendung der Metallverbindungen, hergestellt gemäß einem der Ansprüche 1 bis 14, oder der Einlagerungsverbindungen davon als Pigment für die Herstellung von Laminaten, als Pigment für die Herstellung von Photolacken, als Pigment für die Herstellung von Farbfiltern in Flüssigkristallanzeigen oder als Pigment für den Ink Jet Druck.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 2064093 A
- US 4622391 A
- EP 0994162 A1
- EP 0994163 A1
- EP 0994164 A1
- DE 10328999 A1
- EP 1142960 A
- EP 0074515 A
- EP 0073463 A
- EP 0994163 A
- EP 0994162 A